# EUROPÄISCHE PATENTSCHRIFT

(11) **EP 2 674 623 B1**
(45) Veröffentlichungstag und Bekanntmachung des Hinweises auf die Patenterteilung: **23.09.2015**
(21) Anmeldenummer: 12171534.6
(22) Anmeldetag: 11.06.2012
(51) Int. Cl.: F04B 9/123, F04B 43/04, F04B 43/06, A61M 5/155, A61M 5/142

(54) **Verfahren und Vorrichtung zum dosierten Austrag eines Mediums**
Method and device for metered dispensing of a fluid
Dispositif et procédé destinés à la sortie dosée d'un fluide

(43) Veröffentlichungstag der Anmeldung: 18.12.2013
(73) Patentinhaber: VARTA Microbattery GmbH, 73479 Ellwangen (DE)
(72) Erfinder: Miehlich, Philipp, 73457 Neresheim (DE); Voigt, Uwe, 73457 Essingen (DE)
(74) Vertreter: Patentanwälte Ruff, Wilhelm, Beier, Dauster & Partner

(56) Entgegenhaltungen:
- EP-A1- 0 209 644
- WO-A1-2005/044343
- DE-A1- 2 239 432
- US-A1- 2004 031 695
- US-B1- 6 413 238
- US-B1- 6 520 936

## Beschreibung

Die vorliegende Erfindung betrifft ein Verfahren und eine Vorrichtung zum dosierten Austrag eines Mediums.

Aus dem Stand der Technik ist es bereits seit längerem bekannt, Vorrichtungen zum dosierten Austrag von Medien mittels sogenannter Gaserzeugerzellen zu betreiben, so zum Beispiel aus der deutschen Patentschrift DE 37 11 714 C2. Bei Gaserzeugerzellen handelt es sich bekanntlich um elektrochemische Zellen mit einem positiven und einem negativen Pol, die, wenn ein elektrischer Strom zwischen den Polen fließt, eine zur Menge des fließenden Stroms proportionale Menge eines Gases freisetzen. In der Regel weisen sie eine Ruhespannung auf, worunter zu verstehen ist, dass beim elektrischen Verbinden der Pole ohne externe Triebkraft ein Strom zwischen den Polen fließt und die Gasentwicklung einsetzt. Meist liegen Gasentwicklungszellen in Form von Knopfzellen vor.

Aus der US 6,520,936 B1 ist eine implantierbare Infusionspumpe bekannt, die eine Gasentwicklungszelle umfasst, die von einer ersten expandierbaren Hülle umschlossen ist. Die erste Hülle bildet mit dem Außengehäuse der Pumpe einen mit einem auszutragenden Medium gefüllten Hohlraum, der sich bei Gasentwicklung durch Expansion der ersten Hülle verkleinert und das Medium durch die Öffnung drückt.

Aus der US 6,413,238 B1 ist ein Flüssigkeitsspender in Form einer Spritze bekannt. Die Spritze umfasst einen Druckkolben, der pneumatisch, beispielsweise mit Hilfe einer elektrochemischen Gaserzeugungszelle, bewegt wird. Die Gasmenge wird dabei mit einem gegenüber physikalischen, chemischen oder biologischen Parametern empfindlichen Sensor reguliert.

Die DE 2 239 432 A1 betrifft eine Austragsvorrichtung für Medikamente umfassend einen elektrolytischen Gaserzeuger, der innerhalb eines Medikamentenreservoirs von einer flexiblen Membran umschlossen ist. Bei Gaserzeugung verkleinert sich das Reservoir und das auszutragende Medikament wird durch eine Öffnung gedrückt.

Die WO 2005/044343 A1 betrifft eine Vorrichtung zur dosierten Abgabe von Flüssigkeit mittels einer Gasdruckquelle, die außerhalb einer Druckmittelkammer angeordnet ist. Bei der Gasdruckquelle handelt es sich nicht um eine elektrochemische Zelle.

In der EP 0 209 644 A1 ist eine Austragsvorrichtung für Medikamente umfassend eine elektrochemische Pumpe, die von einem Medikamentenreservoir durch eine gasundurchlässige Membran separiert ist, beschieben. Durch Anlegen einer Spannung zwischen zwei Elektroden wird ein Gas erzeugt, das die Membran in Richtung des Medikamentenreservoirs ausdehnt. Es resultiert ein Medikamentenaustrag durch eine Öffnung.

In der US 2004/0031695 sind elektrochemische N₂-Entwicklungszellen beschrieben, die sich insbesondere als Gaserzeuger in Vorrichtungen zum Austragen von flüssigen Medien eignen.

Problematisch beim Einsatz von Gaserzeugerzellen in Dosiervorrichtungen ist häufig, dass die gasdichte Konstruktion der Vorrichtungen mit einem hohen technischen Aufwand verbunden ist. Dies gilt insbesondere, wenn als Gaserzeugerzelle Wasserstoffentwicklungszellen verwendet werden. Wasserstoffmoleküle sind sehr klein und können selbst durch metallische Barrieren diffundieren.

Der vorliegenden Erfindung lag die Aufgabe zugrunde, eine mittels einer Gaserzeugerzelle betriebene Dosiervorrichtung bereitzustellen, bei der Dichtigkeitsprobleme vermieden werden oder zumindest minimiert sind. Gelöst wird diese Aufgabe durch die Vorrichtung mit den Merkmalen des Anspruchs 1. Bevorzugte Ausführungsformen der erfindungsgemäßen Vorrichtung sind in den abhängigen Ansprüchen 2 bis 11 definiert. Weiterhin ist auch das Verfahren mit den Merkmalen des Anspruchs 12 Gegenstand der vorliegenden Erfindung. Der Wortlaut sämtlicher Ansprüche wird hiermit durch Bezugnahme zum Inhalt der vorliegenden Beschreibung gemacht.

Eine erfindungsgemäße Vorrichtung dient zum dosierten Austrag von Medien, insbesondere von flüssigen oder gasförmigen Medien. Sie umfaßt zwingend die folgenden Komponenten:
- Eine elektrochemische Zelle mit einem positiven und einem negativen Pol, die beim Fließen eines elektrischen Stroms zwischen den Polen eine zur Menge des fließenden Stroms proportionale Menge eines Gases freisetzt.

Besonders bevorzugt können als elektrochemische Zellen Gasentwicklungszellen der in der DE 35 32 335 A1 beschriebenen Bauart zum Einsatz kommen, insbesondere in der wasserstofferzeugenden Ausführungsform. Bevorzugt handelt es sich bei der elektrochemischen Zelle um eine Wasserstoffentwicklungszelle.

Ein Verbindungsmittel, gegebenenfalls einschließlich eines Schalters, über das die Pole der elektrochemischen Zelle miteinander verbunden werden können. Bei dem Verbindungsmittel handelt es sich im einfachsten Fall um einen elektrischen Leiter. In bevorzugten Ausführungsformen kann das Verbindungsmittel einen elektrischen Widerstand umfassen oder aus einem solchen bestehen. Bei dem Widerstand kann es sich grundsätzlich um einen regelbaren Widerstand handeln oder um einen Festwiderstand mit einem im wesentlichen konstanten Ohm-Wert. Bei dem Schalter handelt es sich beispielsweise um einen manuellen oder einen elektronischen Schalter.

Bevorzugt ist gegebenfalls, dass der elektrische Widerstand seine elektrische Leitfähigkeit in Folge eines physikalischen, chemischen oder biologischen Parameters, der direkt auf den Widerstand wirkt oder mittels eines Sensors, der mit dem Widerstand gekoppelt ist, detektiert wird, ändert. So kann er selbst als Schalter fungieren.

Insbesondere ist der Einsatz von temperaturabhängigen Widerständen, beispielsweise von Heißleiter- (NTC-) und Kaltleiter- (PTC-) widerständen möglich. Bekanntlich besteht ein NTC-Widerstand (NTC-Widerstand = Negative Temperature Coefficient Thermistor) aus einem Material, das einen negativen Temperaturkoeffizienten besitzt und somit bei hohen Temperaturen elektrischen Strom besser leitet als bei tiefen Temperaturen. Ein PTC-Widerstand (PTC-Widerstand = Positive Temperature Coefficient Thermistor) besteht dagegen aus einem Material, das einen positiven Temperaturkoeffizienten besitzt und somit bei tiefen Temperaturen elektrischen Strom besser leitet als bei hohen Temperaturen. Mit derartigen Widerständen sind Ausführungformen der erfindungsgemäßen Vorrichtung realisierbar, bei denen eine sich automatisch an die Umgebungstemperatur anpassende Dosierung des auszutragenden Mediums erfolgt.

Weitere bekannte Widerstände, die in Folge der Änderung eines physikalischen Parameters ihre Leitfähigkeit ändern können, sind z.B. LDR-Widerstände (LDR = Light Dependent Resistor), spannungsabhängige Widerstände oder Widerstände, die auf mechanische Belastungen wie Druck, Zug oder Schall reagieren.

Widerstände, die in Abhängigkeit eines chemischen oder biologischen Parameters ihre Leitfähigkeit für elektrisch Strom ändern, sind beispielsweise Widerstände, die auf Luftfeuchtigkeit, die Konzentration eines chemischen Stoffes, insbesondere eines Gases (z.B. Sauerstoff, Wasserstoff, Erdgas), oder einer biologischen Substanz (z.B. eines bakteriellen Keims) reagieren.

Gegebenfalls kann das Verbindungsmittel auch einen regelbaren Widerstand und einen mit diesem gekoppelten Sensor, beispielsweise einem chemischen, biologischen oder physikalischen Sensor, umfassen. Wird von dem Sensor z.B. eine Überschreitung einer vordefinierten Konzentration einer biologischen Substanz oder eines Moleküls (z.B. Sauerstoff) oder einer bestimmten Temperatur oder ein Lichteinfall detektiert, so kann dieser, gegebenfalls über eine geeignete elektronische Schaltung, eine Erniedrigung des regelbaren Widerstands veranlassen. Zwischen den Polen der elektrochemischen Zelle fließt dann ein Strom, dessen Stärke von der detektierten Konzentration, Temperatur oder Lichtstärke abhängt.
- Eine erste, geschlossene Umhüllung, die mindestens teilweise, vorzugsweise vollständig, aus einem flexiblen, für das freigesetzte Gas undurchlässigen Material besteht.

Die erste Umhüllung umschließt die elektrochemische Zelle gasdicht. Von der Zelle generiertes Gas kann nicht aus der ersten Umhüllung entweichen. Entsprechend resultiert aus einer Gaserzeugung eine Volumenexpansion der ersten Umhüllung. Zu diesem Zweck ist die erste Umhüllung bevorzugt aus einem flexiblen Material ausgebildet, insbesondere aus einem dehnbaren, also elastisch oder plastisch verformbaren, Material. Geeignet hierfür sind insbesondere Folien oder Gewebe aus Kunststoff und / oder Metall, insbesondere in Beutelform. Besonders bevorzugt können Verbundmaterialien zum Einsatz kommen, beispielsweise Folien oder Gewebe mit Schichten aus mehreren Materialen, beispielsweise Kunststofffolien mit einer Aluminiumschicht als Sperrschicht für den Wasserstoff. Insbesondere kann die erste Umhüllung auch in Teilen oder vollständig lichtdurchlässig ausgebildet sein.

Erfindungsgemäß ist die erste Umhüllung entweder in einem Hohlraum angeordnet, in dem das auszutragende Medium angeordnet ist und aus dem das Medium über mindestens eine Austragsöffnung ausgetragen werden kann, oder aber sie steht über ein Arbeitsmedium in kommunizierender Verbindung mit einem solchen Hohlraum.

Schwillt die erste Umhüllung in Folge einer Gasentwicklung der elektrochemischen Zelle an, so vergrößert sich ihr Volumen. Ist die erste Umhüllung in dem Hohlraum angeordnet, so verkleinert sich das Volumen des Hohlraums durch die anschwellende erste Umhüllung. Diese übt einen Druck auf das in dem Hohlraum befindliche Medium aus, das in der Folge durch mindestens eine die Begrenzung des Hohlraums durchstoßende Austragsöffnung ausgetragen werden kann. Steht die erste Umhüllung dagegen über ein Arbeitsmedium in kommunizierender Verbindung mit einem Hohlraum, in dem das auszutragende Medium angeordnet ist, so übt die anschwellende erste Umhüllung einen Druck mittelbar über das Arbeitsmedium auf das auszutragende Medium aus.

Bei dem Arbeitsmedium handelt es sich bevorzugt um ein Gas, beispielsweise um Luft, oder eine Flüssigkeit, beispielsweise Wasser oder ein Öl. Der Austrag wird also gegebenenfalls pneumatisch oder hydraulisch bewirkt.

Bevorzugt umfasst die erfindungsgemäße Vorrichtung eine zweite Umhüllung, die die mindestens eine Austragsöffnung aufweist, die erste Umhüllung umschließt und gemeinsam mit dieser den Hohlraum für das auszutragende Medium definiert.

Die mindestens eine Austragsöffnung kann im Grunde nahezu beliebig ausgebildet sein. So kann es sich bei der mindestens einen Austragsöffnung beispielsweise um mindestens eine Pore in der Größenordnung eines aus wenigen Atomen bestehenden Moleküls handeln oder um eine Kanüle, abhängig vom Zweck und entsprechend der konkreten Ausgestaltung der erfindungsgemäßen Vorrichtung.

In einer bevorzugten Ausführungsform der erfindungsgemäßen Vorrichtung ist nicht nur die elektrochemische Zelle innerhalb der ersten Umhüllung angeordnet sondern auch das Verbindungsmittel, gegebenfalls einschließlich des Schalters. Bei dem Schalter handelt es sich in dieser Ausführungsform bevorzugt um einen kontaktlos betätigbaren Schalter. Alternativ sind natürlich auch mechanische Druckschalter einsetzbar. In diesem Fall sollte gegebenenfalls nicht nur die erste Umhüllung flexibel ausgebildet sein sondern mindestens auch ein Teil der zweiten Umhüllung, damit eine Betätigung des Druckschalters möglich ist.

Insbesondere, wenn Widerstände eingesetzt werden, deren elektrische Leitfähigkeit in Folge eines physikalischen, chemischen oder biologischen Parameters, der direkt auf den Widerstand wirkt, sich ändert, oder die regelbar sind und mit einem der erwähnten chemischen, biologischen oder physikalischen Sensoren gekoppelt sind, kann es allerdings auch bevorzugt sein, dass der Widerstand und/oder der Sensor außerhalb der ersten Umhüllung angeordnet ist, gegebenfalls auch außerhalb der zweiten Umhüllung. Für das Verbindungsmittel sind in dieser Ausführungsform entsprechende Durchlässe durch die erste Umhüllung, gegebenfalls auch durch die zweite Umhüllung, vorgesehen.

Die zweite Umhüllung besteht bevorzugt zumindest teilweise, vorzugsweise vollständig, aus einer Folie oder aus einem Gewebe. Das Material aus dem die zweite Umhüllung besteht, muss im Gegensatz zu dem der ersten Umhüllung nicht zwingend gasdicht sein. Idealerweise sollte die zweite Umhüllung allerdings aus einem nicht dehnbaren Material bestehen, so dass ein Druckausgleich innerhalb der zweiten Umhüllung in Folge einer Volumenexpansion der ersten Umhüllung ausschließlich durch den gewünschten Auftrag des Mediums stattfinden kann. So kann es sich bei der zweiten Umhüllung beispielsweise um ein Gehäuse aus einem festen Material, beispielsweise aus einem Metallblech, Glas oder aus einem Kunststoffgussteil, handeln.

Auch die zweite Umhüllung kann gegebenfalls in Teilen oder vollständig transparent ausgebildet sein.

Der Hohlraum zwischen der ersten und der zweiten Umhüllung ist mit einem porösen elastischen Material, insbesondere mit einem Schwamm, befüllt, welches mit dem auszutragenden Medium beladen ist. Dieses Material kann mit dem auszutragenden Medium beispielsweise getränkt werden und dieses speichern.

Alternativ kann das Medium auch in flüssiger, gelartiger oder pastenartiger oder fester Form in dem Hohlraum angeordnet sein.

Bevorzugt handelt es sich bei dem Medium um ein Schmiermittel, ein Desinfektionsmittel, einen Duftstoff oder einen pharmazeutischen und/oder medizinischen Wirkstoff. Auch antibakterielle Mittel, antivirale Mittel, Reinigungsmittel, Haft- und Klebemittel in grundsätzlich jedweder Form und Zusammensetzung können mittels der erfindungsgemäßen Dosiervorrichtung ausgetragen werden.

Losgelöst von einer konkreten Ausführungsform der erfindungsgemäßen Vorrichtung ist das erfindungsgemäße Verfahren zum dosierten Austrag eines solchen Mediums.

Erfindungsgemäß wird zur Bewirkung des Austrags eine gaserzeugende elektrochemische Zelle in Betrieb genommen, die innerhalb einer geschlossenen, für das erzeugte Gas undurchlässigen Umhüllung angeordnet ist. In Übereinstimmung mit obigen Ausführungsformen schwillt in Folge der Gaserzeugung die Umhüllung an, wodurch das Volumen des Hohlraumes reduziert oder über ein Arbeitsmedium Druck auf das in dem Hohlraum angeordnete poröse elastische Material, welches mit dem auszutragenden Medium beladen ist, ausgeübt wird. Dadurch entsteht ein Druck, der auf das in dem Hohlraum enthaltene Medium wirkt, welches in der Folge aus dem Hohlraum ausgetragen wird.

Weitere Merkmale und auch Vorteile der vorliegenden Erfindung ergeben sich aus der nun folgenden Beschreibung bevorzugter Ausführungsformen der erfindungsgemäßen Vorrichtung in Verbindung mit den Unteransprüchen. Die dargestellten Merkmale der erfindungsgemäßen Vorrichtung können jeweils für sich oder zu mehreren in Kombination bei einer Ausführungsform der Erfindung verwirklicht sein. Die Ausführungsbeispiele dienen lediglich zur Erläuterung und zum besseren Verständnis der Erfindung und sind in keiner Weise einschränkend zu verstehen.

### Figurenbeschreibung:

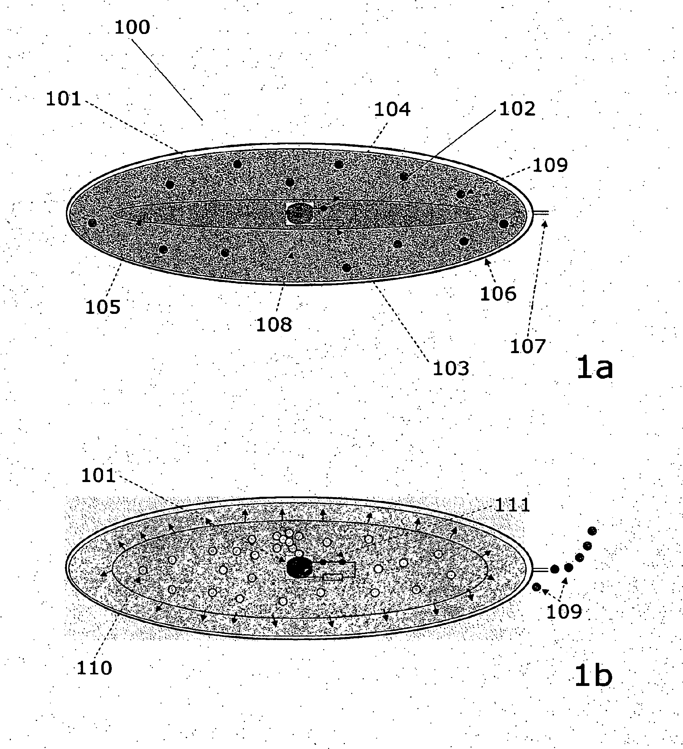

- Fig. 1a: zeigt schematisch eine Ausführungsform einer erfindungsgemäßen Vorrichtung vor ihrer Aktivierung.
- Fig. 1b: zeigt die gleiche Ausführungsform der erfindungsgemäßen Vorrichtung nach ihrer Aktivierung.

Dargestellt ist die Dosiervorrichtung **100.** Diese umfasst die Gaserzeugungszelle **101,** deren Pole mittels des Verbindungsmittels 102 kurzeschlossen bzw. elektrisch miteinander verbunden werden können. Das Verbindungsmittel **102** umfasst den elektrischen Widerstand **103** sowie den Schalter **104.** Bei dem Widerstand **103** kann es sich beispielsweise um einen NTC-, PTC- oder LDR-Widerstand handeln. Bei dem Schalter **104** kann es sich beispielsweise um einen Mikro-Foliendruckschalter handeln. Die Gaserzeugungszelle **101** und das Verbindungsmittel **102** sind innerhalb der ersten Umhüllung **105** angeordnet. Diese umschließt die erstgenannten Komponenten gasdicht. Bei der Umhüllung **105** handelt es sich beispielsweise um einen Beutel aus einem Kunststoff-Aluminium-Verbundmaterial. Dieser ist wiederum innerhalb der zweiten Umhüllung **106** ausgebildet, die eine Austrittsöffnung **107** aufweist. Gemeinsam definieren die zweite Umhüllung **106** und die erste Umhüllung **105** einen Hohlraum **108,** der vorliegend mit einem schwammartigen Trägermaterial, der mit dem auszutragenden Medium **109** beladen ist.

Bei Inbetriebnahme der Gaserzeugungszelle **101** durch Schließen des Schalters **104** wird von der Gaserzeugsungszelle **101** ein Gas **110** erzeugt. In der Folge dehnt sich die erste Umhüllung **105** aus, wodurch der Hohlraum **108** zwischen der ersten Umhüllung und der zweiten Umhüllung verkleinert wird. Der dabei auf das schwammartige Trägermaterial **111** wirkende Druck bewirkt einen Austritt des auszutragenden Mediums **109** über die Auftragsöffnung **107.**

## Patentansprüche

1. Vorrichtung zum dosierten Austrag eines Mediums, umfassend
• eine elektrochemische Zelle (101) mit einem positiven und einem negativen Pol, die beim Fließen eines elektrischen Stroms zwischen den Polen eine zur Menge des fließenden Stromes proportionale Menge eines Gases (110) freisetzt,
• ein Verbindungsmittel (102), gegebenenfalls einschließlich eines Schalters (104), über das die Pole der elektrochemischen Zelle (101) miteinander verbunden werden können,
• eine erste, geschlossene Umhüllung (105) zumindest teilweise bestehend aus einem flexiblen, für das freigesetzte Gas undurchlässigen Material, die die elektrochemische Zelle (101) gasdicht umschließt,
wobei die erste Umhüllung (105) in einem Hohlraum (108) angeordnet ist, in dem das auszutragende Medium (109) angeordnet ist und aus dem das Medium (109) über mindestens eine Austragsöffnung (107) ausgetragen werden kann oder die erste Umhüllung (105) über ein Arbeitsmedium in kommunizierender Verbindung mit einem solchen Hohlraum (108) steht, **dadurch gekennzeichnet, dass** der Hohlraum (108) mit einem porösen elastischen Material befüllt ist, welches mit dem auszutragenden Medium (109) beladen ist.

2. Vorrichtung nach Anspruch 1, **dadurch gekennzeichnet, dass** sie eine zweite Umhüllung (106) umfasst, die die mindestens eine Austragsöffnung (107) aufweist, die erste Umhüllung (105) umschließt und gemeinsam mit dieser den Hohlraum (108) bildet.

3. Vorrichtung nach Anspruch 1 oder Anspruch 2, **dadurch gekennzeichnet, dass** die elektrochemische Zelle (101) eine Wasserstoffentwicklungszelle ist.

4. Vorrichtung nach einem der vorhergehenden Ansprüche, **dadurch gekennzeichnet, dass** das Verbindungsmittel (102) einen elektrischen Widerstand (103) umfasst.

5. Vorrichtung nach einem der vorhergehenden Ansprüche, **dadurch gekennzeichnet, dass** es sich bei dem elektrischen Widerstand (103) um einen Festwiderstand mit einem im Wesentlichen konstanten Ohm-Wert oder um einen regelbaren Widerstand handelt.

6. Vorrichtung nach einem der vorhergehenden Ansprüche, **dadurch gekennzeichnet**, der elektrische Widerstand (103) seine elektrische Leitfähigkeit in Folge eines physikalischen, chemischen oder biologischen Parameters, der direkt auf den Widerstand wirkt oder mittels eines Sensors, der mit dem Widerstand gekoppelt ist, detektiert wird, ändert.

7. Vorrichtung nach einem der vorhergehenden Ansprüche, **dadurch gekennzeichnet, dass** die erste Umhüllung (105) aus einer Folie oder aus einem Gewebe besteht.

8. Vorrichtung nach einem der vorhergehenden Ansprüche, **dadurch gekennzeichnet, dass** das Verbindungsmittel (102), vorzugsweise einschließlich des Schalters (104), innerhalb der ersten Umhüllung (105) angeordnet ist.

9. Vorrichtung nach einem der vorhergehenden Ansprüche, **dadurch gekennzeichnet, dass** die zweite Umhüllung (106) zumindest teilweise aus einem flexiblen Material, insbesondere aus einer Folie oder aus einem Gewebe, besteht.

10. Vorrichtung nach einem der vorhergehenden Ansprüche, **dadurch gekennzeichnet, dass** der Hohlraum (108) mit einem Schwamm befüllt ist, welcher mit dem auszutragenden Medium (109) beladen ist.

11. Vorrichtung nach einem der vorhergehenden Ansprüche, **dadurch gekennzeichnet, dass** das auszutragende Medium (109) ein Schmiermittel, ein Desinfektionsmittel, ein Duftstoff oder ein pharmazeutischer Wirkstoff ist.

12. Verfahren zum dosierten Austrag eines Mediums (109), das innerhalb eines Hohlraums (108) angeordnet ist, der mit einem porösen elastischen Material befüllt ist, welches mit dem auszutragenden Medium (109) beladen ist, wobei zur Bewirkung des Austrags eine gaserzeugende elektrochemische Zelle (102) in Betrieb genommen wird, die innerhalb einer geschlossenen, für das erzeugte Gas undurchlässigen Umhüllung (105) angeordnet ist und durch die in Folge der Gaserzeugung anschwellende Umhüllung (105) das Volumen des Hohlraumes (108) reduziert oder über ein Arbeitsmedium Druck auf das in dem Hohlraum (108) angeordnete auszutragende Medium (109) ausgeübt wird.

## Claims

1. Device for metered discharge of a medium, comprising
• an electrochemical cell (101) including a positive and a negative pole, which
during flow of an electrical current between the poles releases an amount of gas (110) proportional to the amount of current flowing,
• a connecting means (102), as required including a switch (104), by which the poles of the electrochemical cell (101) can be connected to each other,
• a first closed casing (105) at least partially composed of a material which is flexible and impermeable for the released gas to enclose the electrochemical cell (101) in a gas-tight manner,
wherein the first casing (105) is arranged in a cavity (108) in which the medium (109) to be discharged is disposed and from which the medium (109) can be discharged via at least one discharge opening (107) or the first casing (105) is in communicating connection with such a cavity (108) via a working medium,
**characterized in that**
the cavity (108) is filled with a porous elastic material which is loaded with the medium (109) to be discharged.

2. Device according to claim 1, **characterized in that** it comprises a second casing (106) which includes the at least one discharge opening (107), encloses the first casing (105) and together with the latter constitutes the cavity (108).

3. Device according to claim 1 or claim 2, **characterized in that** the electrochemical cell (101) is a hydrogen producing cell.

4. Device according to any one of the preceding claims, **characterized in that** the connecting means (102) comprises an electrical resistor (103).

5. Device according to any one of the preceding claims, **characterized in that** the electrical resistor (103) is a fixed resistor having an essentially constant ohmic value or is an adjustable resistor.

6. Device according to any one of the preceding claims, **characterized in that** the electrical resistor (103) varies the electrical conductivity thereof in response to a physical, chemical or biological parameter which acts on the resistor directly or is detected by means of a sensor which is coupled to the resistor.

7. Device according to any one of the preceding claims, **characterized in that** the first casing (105) is composed of a film or of a fabric.

8. Device according to any one of the preceding claims, **characterized in that** the connecting means (102) is arranged within the first casing (105), preferably together with the switch (104).

9. Device according to any one of the preceding claims, **characterized in that** the second casing (106) is at least partially composed of a flexible material, in particular of a film or of a fabric.

10. Device according to any one of the preceding claims, **characterized in that** the cavity (108) is filled with a sponge which is loaded with the medium (109) to be discharged.

11. Device according to any one of the preceding claims, **characterized in that** the medium (109) to be discharged is a lubricant, a disinfectant, a fragrance or a pharmaceutical active agent.

12. Method for metered discharge of a medium (109) which is disposed within a cavity (108) filled with a porous elastic material which is loaded with the medium (109) to be discharged, wherein for causing the discharge a gas-generating electrochemical cell (101) is put in operation, which cell is arranged within a closed casing (105), the casing being impermeable for the generated gas, and wherein the volume of the cavity (108) is reduced due to the casing (105) expanding as a result of the gas generation or pressure is applied to the medium (109) to be discharged present within the cavity (108) via a working medium.

## Revendications

1. Dispositif destiné à la distribution dosée d'un fluide, comprenant :
- une cellule électrochimique (101) comprenant un pôle positif et un pôle négatif, qui libère, lors de la circulation d'un courant électrique entre les pôles, une quantité d'un gaz (110) proportionnelle à la quantité du courant circulant,
- un moyen de connexion (102), éventuellement incluant un commutateur (104), par le biais duquel les pôles de la cellule électrochimique (101) peuvent être connectés l'un à l'autre,
- une première enveloppe fermée (105) constituée au moins en partie d'un matériau flexible, imperméable au gaz libéré, qui entoure de manière étanche aux gaz la cellule électrochimique (101),
la première enveloppe (105) étant disposée dans une cavité (108) dans laquelle est disposé le fluide à distribuer (109), et hors de laquelle le fluide (109) peut être distribué par le biais d'au moins une ouverture de distribution (107) ou la première enveloppe (105) étant en liaison de communication par le biais d'un fluide de travail avec une telle cavité (108), **caractérisé en ce que** la cavité (108) est remplie d'un matériau élastique poreux qui est chargé avec le fluide (109) à distribuer.

2. Dispositif selon la revendication 1, **caractérisé en ce qu'**il comprend une deuxième enveloppe (106) qui présente l'au moins une ouverture de distribution (107), qui entoure la première enveloppe (105) et qui forme conjointement avec celle-ci la cavité (108).

3. Dispositif selon la revendication 1 ou la revendication 2, **caractérisé en ce que** la cellule électrochimique (101) est une cellule de dégagement d'hydrogène.

4. Dispositif selon l'une quelconque des revendications précédentes, **caractérisé en ce que** le moyen de connexion (102) comprend une résistance électrique (103).

5. Dispositif selon l'une quelconque des revendications précédentes, **caractérisé en ce que** la résistance électrique (103) est une résistance fixe avec une valeur ohmique essentiellement constante ou une résistance réglable.

6. Dispositif selon l'une quelconque des revendications précédentes, **caractérisé en ce que** la résistance électrique (103) modifie sa conductibilité électrique en fonction d'un paramètre physique, chimique ou biologique, qui agit directement sur la résistance ou qui est détecté au moyen d'un capteur accouplé à la résistance.

7. Dispositif selon l'une quelconque des revendications précédentes, **caractérisé en ce que** la première enveloppe (105) se compose d'un film ou d'un tissu.

8. Dispositif selon l'une quelconque des revendications précédentes, **caractérisé en ce que** le moyen de connexion (102), de préférence incluant le commutateur (104), est disposé à l'intérieur de la première enveloppe (105).

9. Dispositif selon l'une quelconque des revendications précédentes, **caractérisé en ce que** la deuxième enveloppe (106) se compose au moins en partie d'un matériau flexible, en particulier d'un film ou d'un tissu.

10. Dispositif selon l'une quelconque des revendications précédentes, **caractérisé en ce que** la cavité (108) est remplie d'une éponge qui est chargée avec le fluide (109) à distribuer.

11. Dispositif selon l'une quelconque des revendications précédentes, **caractérisé en ce que** le fluide (109) à distribuer est un lubrifiant, un désinfectant, un parfum ou une substance active pharmaceutique.

12. Procédé destiné à la distribution dosée d'un fluide (109), qui est disposé à l'intérieur d'une cavité (108) qui est remplie d'un matériau élastique poreux qui est chargé avec le fluide (109) à distribuer, une cellule électrochimique (102) produisant du gaz étant mise en service pour réaliser la distribution, laquelle est disposée à l'intérieur d'une enveloppe fermée (105) imperméable au gaz produit, et, par le gonflement de l'enveloppe (105) sous l'effet de la production de gaz, le volume de la cavité (108) étant réduit ou une pression étant exercée par le biais d'un fluide de travail sur le fluide (109) à distribuer disposé dans la cavité (108).
